# EUROPEAN PATENT SPECIFICATION

(11) **EP 1 095 651 B1**
(45) Date of publication and mention of the grant of the patent: **03.01.2007**
(21) Application number: 00850162.9
(22) Date of filing: 11.10.2000
(51) Int. Cl.: A61K 9/28

(54) **Coated tablets having minimised susceptability to food interferance.**
Filmtabletten mit minimierter Abhängigkeit von Nahrungsmitteleinfluss Anfälligkeit
Comprimes enrobes ayant suceptibilité de interférance alimentaire reduite au minimum

(30) Priority: 28.10.1999 SE 9903879
(43) Date of publication of application: 02.05.2001
(73) Proprietor: Watson Laboratories, Inc., Salt Lake City UT 84108 (US)
(72) Inventor: Fyhr, Peter, 277 56 Brösarp (SE); Corselli, Marta, 216 22 Malmö (SE); Waxegard, Staffan, 212 28 Malmö (SE)
(74) Representative: Jakobsen, Gert Hoey

(56) References cited:
- EP-A- 0 335 560
- EP-A- 0 601 508
- US-A- 4 557 925
- US-A- 4 610 870

## Description

### FIELD OF THE INVENTION

The present invention concerns a controlled-release pharmaceutical preparation. Specifically the invention concern controlled-release tablets protected from food interference.

### BACKGROUND OF THE INVENTION

Innumerable types of controlled-release tablets are known, which have had greater or lesser degrees of success accomplishing the desired result, namely the attainment of a substantially constant and controlled dissolution rate during a major portion of their dissolution time when exposed to gastrointestinal fluids.

One such tablet is disclosed in US 4,557,925. Medical preparations developed according to this patent are currently used and well accepted by the patients. However, in some cases it has been found that the release of the pharmacologically active substance can be disturbed by the food intake, a consequence of which is that the constant release rate is disturbed. The tablet comprises a drug-containing tablet core and a water-insoluble coating or membrane surrounding the same, wherein the coating or membrane contains a pore-creating substance. Without being bound to any specific theory it is believed that the dietary lipids are adsorbed onto or absorbed into the hydrophobic coating or membrane thereby preventing the release of the drug.

### OBJECT OF THE INVENTION

A main object of the present invention is to reduce or eliminate this disadvantage of this and similar pharmaceutical preparations.

### SUMMARY OF THE INVENTION

The invention concerns an essentially zero-order controlled-release pharmaceutical tablet comprising a drug-containing tablet core surrounded by a first coating or membrane essentially consisting of a water insoluble polymer having fine water soluble particles randomly distributed therein and a second hydrophilic film coating essentially consisting of a pharmaceutically acceptable water insoluble polymer selected from ethylcellulose, methylcellulose, polymetacrylate, polyvinylacetate, poly(vinylacetate-co-crotonic acid), poly(vinylacetateco-vinyl alcohol), poly(ethyl acrylate, methyl methacrylate, trimethylammonioethyl methacrylate chloride) and a pharmaceutically acceptable water soluble polymer selected from guar gum, hydroxyethylcellulose, hydroxypropylcellulose, hydroxypropylmethylcellulose, polyethyleneoxide, polyvinylalcohol, polyvinylpyrrolidone. By providing this second coating it has been found that a food neutral system is created that will allow the drug to be released at an essentially zero order release rate in the presence of food. The invention also concerns a method for the preparation of such a pharmaceutical tablet.

Controlled release pharmaceutical preparations having an outer porous coating consisting of a hydrophobic polymeric substance or a hydrophobic polymeric substance and a hydrophilic polymeric substance are disclosed in EP 335 560. In these preparations the controlled release is achieved by the coating, the porosity of which is achieved, not by water soluble particles as in the present invention, but by a proper selection of the polymer substances of the coating. Nothing is mentioned about food interference problems and how to eliminate such problems.

### DETAILED DESCRIPTION OF THE INVENTION

More specifically the second hydrophilic film coating consists essentially of a pharmaceutically acceptable water insoluble polymer such as ethylcellulose, methylcellulose, polymetacrylate, polyvinylacetate, poly(vinylacetate-co-crotonic acid), poly(vinylacetateco-vinyl alcohol), poly(ethyl acrylate, methyl methacrylate, trimethylammonioethyl methacrylate chloride) and a pharmaceutically acceptable water soluble polymer such as guar gum, hydroxyethylcellulose, hydroxypropylcellulose, hydroxypropylmethylcellulose, polyethyleneoxide, polyvinylalcohol, polyvinylpyrrolidone. Especially preferred are mixtures of cellulose polymers such as ethylcellulose, hydroxypropyl methylcellulose, preferably at a concentration of 50-90 % ethylcellulose and 10-50 % hydroxypropyl methylcellulose. Preferably the hydrophilic film coating also includes a plasticizer. Examples of plastizicers are triethyl citrate, acetylbutyl citrate and polyethyleneoxide. A preferred plastizicer is acetyl tributyl citrate.

According to one embodiment of the invention a sub coating may be provided between the first hydrophobic coating or membrane and the second hydrophilic film coating. This sub or intermediate coating may be selected from the group consisting of guar gum, hydroxyethylcellulose, hydroxypropylcellulose, hydroxypropylmethylcellulose, polyethyleneoxide, polyvinylalcohol. A preferred sub coating may consist of polyvinylpyrrolidone.

The water insoluble, microporous coating or membrane may be of the type disclosed in the US patent 4 557 925 referred to above and may consist of cellulosa derivatives, acrylic polymers, and other high molecule polymers such as ethyl cellulose, cellulose acetate, cellulose propionate, cellulose butyrate, cellulose valerate, cellulose acetate propionate, polyvinylacetate, polyvinylalcohol, polyvinylchloride, polyvinyl formal, polyvinyl butyral, ladder polymer of sesquiphenyl siloxane, polymethyl methacrylate, polycarbonate, polyester, coumaroneindene polymer, polybutadiene, vinyl chloride-vinyl acetate copolymer, ethylene-vinyl acetate copolymer and vinyl chloridepropylene-vinyl acetate terpolymer, and a water-soluble pore-creating substance. The pore-creating substance is a water-soluble substance selected from the group consisting of saccharose, sodium chloride and potassium chloride, or selected from the group consisting of polyvinylpyrrolidone and a polyethyleneglycol.

The drug in the core could for example be tranquillizers, hypnotics, antibiotics, antihypertensives, antianginas, analgesics, antiinflamatories, neuroleptics, antidiabetic, diuretics, antikolinergics, antihyperacidics or antiepileptics. The drug substance could for example be potassium chloride, theophylline, a theophylline salt, phenylpropanolamine, sodium salicylate, choline theophyllinate, paracetamole, carbidopa, levodopa, diltiazem, enalapril, verapamil, naproxen, pseudoephedrin, nicorandil, oxybutyin, morphine, oxycodone or propranolol. One embodiment of the invention concerns a preparation containing Naproxen as the drug active substance in the tablet core. The solubility of the drug active substance is preferably more than 10 mg/l.

The present invention also provides a method of preparing this controlled-release preparation comprising the steps of; dissolving the coating or membrane polymer (poly-(vinylchloride-co-vinylalcohol-co-vinylacetate)) in a solvent, preparing a suspension of the pore-creating material, dissolving the sub coating agent in a solvent, dissolving the film-polymer in a solvent, providing a pharmaceutical tablet combining the suspension of pore-creating material and solution of the coating- or membrane-polymer to form a coating fluid, applying the coating fluid in the form of a suspension on the tablet and drying the coating fluid on the tablet, applying the dissolved sub coating agent to the tablet and drying the tablet, applying the dissolved film-polymer to the coated tablet and drying the tablet, to provide a coated tablet having water-soluble pore-creating material randomly distributed within the first coating- or membrane-polymer, covered by a sub coating and a second film-polymer.

The present invention is not limited in either its film-coated controlled release tablet aspect or in its method aspect by the drug or type of drug incorporated in the tablet. Any tablet containing any presently known or future discovered orally acting drug may be coated according to the present invention, to provide the highly advantageous controlled release pharmaceutical tablets of the present invention that physically prevent interference between the tablet and lipids in the gastrointestinal fluids.

The invention is further illustrated by, but should not be limited to, the following preparation and example.

### Example

| Constituents: | | | |
|---|---|---|---|
| Quantity | | | |
| | mg/tablet | | |
| Core | | | |
| Naproxen Sodium | | 548** | |
| Sodium Phosphate, | | | |
| dibasic heptahydr. | | 38,9 | |
| Lactose 125M | | 49,1 | |
| Povidone, Kollidon 25 | | 52,0 | |
| Magnesium Stearate | | 10,3 | |
| Ethanol 99,5%* | | | |
| 75,0 | | | |
| Weight of core: | | 698 | |

| Membrane coating (First coating) | | | |
|---|---|---|---|
| Sucrose, powder | | 57,1 | |
| Coating polymer | | 9,77 | |
| Acetyl Tributyl Citrate | | 1,74 | |
| Castor Oil, polymerized | | 1,33 | |
| Sodium Hydrogen Carbonate | 2,09 | | |
| Acetone* | | | 542 |
| Weight of the membrane coating: | 72,0 | | |

| Sub coating | | | |
|---|---|---|---|
| polyvinylpyrrolidone | | 11,4 | |
| Ethanol 99,5%* | | | 123 |
| Weight of the sub coating: | 11,4 | | |

| Film coating (Second coating) | | | |
|---|---|---|---|
| Ethylcellulose N7 | | 7,00 | |
| Hydroxypropyl Methylcellulose | | 3,00 | |
| Acetyl Tributyl Citrate (ATBC | | 2,00 | |
| Ethanol 99,5%* | | | 161 |
| Water, purified* | | 27,0 | |
| Weight of the film coating: | 12,0 | | |
| Total weight of the tablet: | 793 | | |

| | | | |
|---|---|---|---|
| Note: The weight of the coating may vary +/- 10%. * Evaporates during the process ** Equivalent to 500 mg Naproxen | | | |

### A. Core

Dibasic sodium phosphate heptahydrate is sieved through a 0,5 mm screen in a Quadro mill. Naproxen sodium, lactose, Povidone and the milled buffering agent are sieved through a 1 mm screen (Quadro) into an intensive mixer for wet granulation. The substances are granulated with ethanol.

The granulation is dried in a ventilated air chamber at 40°C for approx. 2 hours. Loss on drying limits: 2.5-3.5%.

The dried granulation and magnesium stearate are sieved through a lmm screen (Quadro) and transferred into a double cone tumbling mixer and final mixing is then performed.

Tabletting is achieved on a rotary tablet press.

### B. Coating processes

The tablet cores are coated in several steps:
- Membrane coating (the rate limiting step)
- Sub coating (to separate the membrane coating from the film coating)
- Film coating (to prevent a negative food effect)
- Drying in the drying vessels

### B.1. Membrane coating process

Preparation of the membrane coating suspension

### The coating suspension is manufactured in a two-step procedure (I-II):

### I. Concentrated suspension

1. The sucrose powder and sodium hydrogen carbonate are dispersed in acetone under continuous agitation. The agitated suspension is milled together with additional acetone, acetyl tributyl citrate and polymerized castor oil in a pearl mill. The solution is transferred into a final mixing vessel, where it is kept under continuous agitation.
2. The coating polymer is dissolved in acetone. The solution is transferred into the final mixing vessel.
3. The concentrated suspension is kept under continuous agitation in the final mixing vessel until it is used.

### II. Coating suspension

1. The concentrated suspension is transferred into a vessel included in the coating equipment and diluted with acetone.
2. The coating suspension is kept under continuous agitation during the membrane coating process.

### Membrane coating/drying in the pan

1. The cores are transferred into an Accela Cota, equipped with a hot air source and exhaust device.
2. The hot air temperature is 57°C.
3. The coating suspension is sprayed onto the cores using an airborne spray device.
4. After membrane coating the tablets are dried in the coating pan for 15 minutes at 57°C.

### B.2. Sub coating process

1. Ethanol and povidone are added to the stainless steel vessel during stirring for about 10 minutes.
2. The sub coating is performed in the Accela Cota immediately after the membrane coating/drying.
3. After sub coating the tablets are dried in the pan for 5 minutes at 57°C.

### B.3. Film coating process

1. Ethylcellulose is dissolved in ethanol in the stainless steel vessel during hard stirring for about 10 minutes.
2. Hydroxypropyl methylcellulose and acetyl tributyl citrate are added to the vessel during continued stirring for about 10 minutes.
3. Water is added to the vessel during hard stirring. Stirring continued for about 10 minutes.
4. The film coating is performed in the Accela Cota immediately after the sub coating and drying.
5. After film coating the tablets are dried in the coating pan for 10 minutes at 57°C.

### B.4. Drying in the drying vessels

After final coating the tablets are dried for about 16 hours at 40°C in a ventilated drying container.

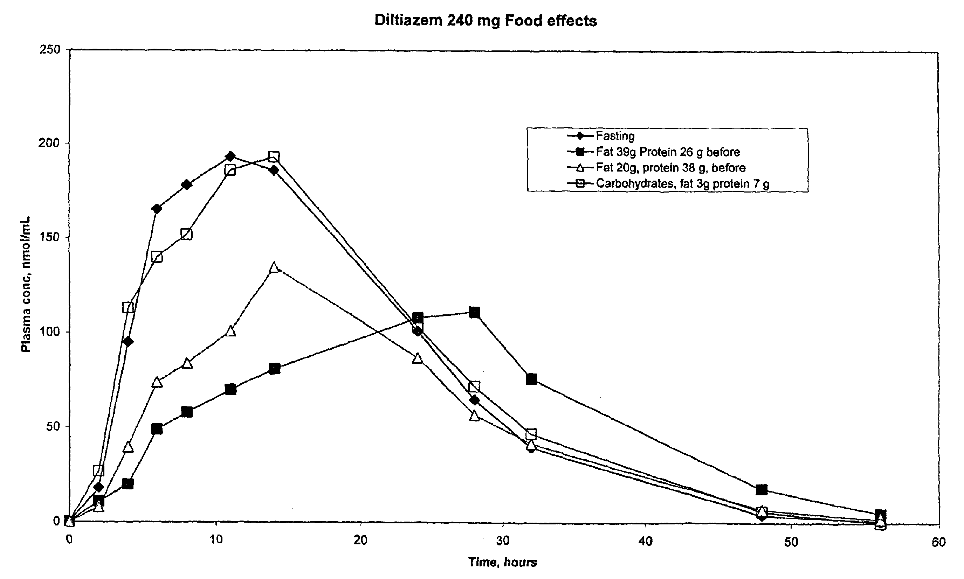

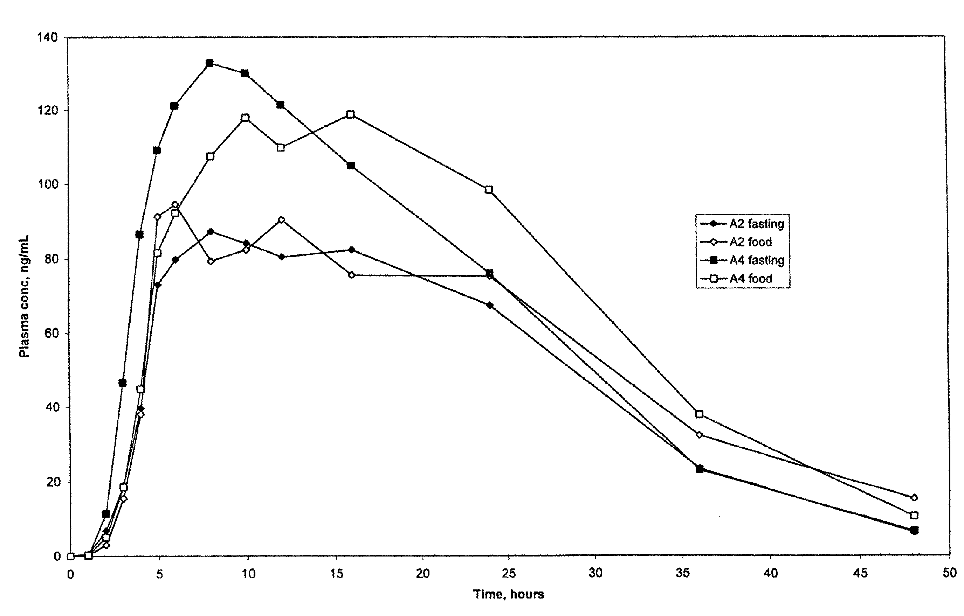

The effect of food containing different amounts of fat on a tablet without the second coating is clearly demonstrated in Figure 1. In figure 2 the same tablets have been provided with a second coating according to the present invention which coating has been applied on the first coating. It is clearly seen that the food interference is essentially eliminated by the second coating.

## Claims

1. A pharmaceutical tablet comprising a drug-containing tablet core surrounded by a first coating or membrane providing an essentially zero-order controlled-release of the drug, said coating or memebrane essentially consisting of a water insoluble polymer having fine water soluble particles randomly distributed therein, and a second coating, **characterised in that** the second coating is a hydrophilic film coating, essentially consisting of a pharmaceutically acceptable water insoluble polymer selected from ethylcellulose, methylcellulose, polymetacrylate, polyvinylacetate, poly(vinylacetate-co-crotonic acid), poly(vinylacetateco-vinyl alcohol), poly(ethyl acrylate, methyl methacrylate, trimethylammonioethyl methacrylate chloride) and a pharmaceutically acceptable water soluble polymer selected from guar gum, hydroxyethylcellulose, hydroxypropylcellulose, hydroxypropylmethylcellulose, polyethyleneoxide, polyvinylalcohol, polyvinylpyrrolidone, for eliminating or minimising food interference.

2. The tablet according to claim 1, **characterised in that** the second film coating is a mixture of ethyl cellulose and hydroxypropyl methylcellulose.

3. The tablet according to claim 1 or 2, **characterised in that** the second film coating contains 50-90% ethyl cellulose and 10-50% hydroxypropyl methylcellulose.

4. The tablet according to any one of the claims 1-3, **characterised in that** a sub coating is provided between the first and the second coating.

5. The tablet according to claim 4, **characterised in that** the sub coating consists of polyvinylpyrrolidone.

6. The tablet according to any of the claims 1-5, **characterised in that** the first coating essentially consists of at least one terpolymer having a water soluble pore creating agent dispersed therein.

7. The tablet according to any of the claims 1-6, **characterised in that** the first coating essentially consists of a terpolymer of vinylchloride, vinylacetate and vinylalcohol.

8. The tablet according to any of the claims 1-7, **characterised in that** the drug in the core is selected from the group consisting of tranquillizers, antibiotics, hypnotics, antihypertensives, antianginas, analgesics, antiinflamatories, neuroleptics, antidiabetic, diuretics, antikolinergics, antihyperacidics or antiepileptics.

9. The tablet according to any of the claims 1-8, **characterised in that** the drug in the core is potassium chloride, theophylline, a theophylline salt, phenylpropanolamine, sodium salicylate, choline theophyllinate, paracetamole, carbidopa, levodopa, diltiazem, enalapril, verapamil, naproxen, pseudoephedrin, nicorandil, oxybutuin, morphine, oxycodone or propranolol.

10. The tablet according to any of the claims 1-9 **characterised in that** the drug in the core is Naproxen.

11. A method of preparing the tablet according to claim 1, comprising the steps of;
a) dissolving the coating- or membrane-polymer in a solvent,
b) preparing a suspension of the pore-creating material,
c) dissolving the sub coating agent in a solvent,
d) dissolving the film-polymer in a solvent,
e) providing a pharmaceutical tablet, combining the suspension of pore-creating material and solution of the coating- or membrane-polymer to form a coating fluid,
f) applying the coating fluid in the form of a suspension onto the tablet and drying the coating fluid on the tablet,
g) applying the dissolved sub coating agent to the tablet and drying the tablet,
h) applying the dissolved film-polymer to the coated tablet and drying the tablet, to provide a coated tablet having water-soluble pore-creating material randomly distributed within the first coating- or membrane-polymer, covered by a sub coating and a second film-polymer coating.

12. Use of a hydrophilic film as a second tablet coating for eliminating or minimising food interference of a pharmaceutical tablet comprising a drug-containing tablet core surrounded by a first coating or membrane providing an essentially zero-order controlled-release of the drug, said coating or membrane essentially consisting of a water insoluble polymer having fine water soluble particles randomly distributed therein.

## Patentansprüche

1. Pharmazeutische Tablette, die einen wirkstoffhaltigen Tablettenkern umfasst, der umgeben ist von einer ersten Beschichtung oder Membran, die für eine gezielte Freisetzung des Wirkstoffs mit im Wesentlichen nullter Ordnung sorgt, wobei die Beschichtung oder Membran im Wesentlichen aus einem wasserunlöslichen Polymer besteht, in dem feine wasserlösliche Teilchen statistisch verteilt sind, und einer zweiten Beschichtung, **dadurch gekennzeichnet, dass** die zweite Beschichtung eine hydrophile Filmbeschichtung ist, die im Wesentlichen aus einem pharmazeutisch annehmbaren wasserunlöslichen Polymer, das aus Ethylcellulose, Methylcellulose, Polymethacrylat, Polyvinylacetat, Poly(vinylacetat-co-krotonsäure), Poly-(vinylacetat-co-vinylalkohol) und Poly(ethylacrylat-methylmethacrylattrimethylammonioethylmethacrylatchlorid) ausgewählt ist, und einem pharmazeutisch annehmbaren wasserlöslichen Polymer, das aus Guarkernmehl, Hydroxyethylcellulose, Hydroxypropylcellulose, Hydroxypropylmethylcellulose, Polyethylenoxid, Polyvinylalkohol und Polyvinylpyrrolidon ausgewählt ist, besteht, zur Beseitigung oder Minimierung von Störungen durch die Nahrung.

2. Tablette gemäß Anspruch 1, **dadurch gekennzeichnet, dass** die zweite Filmbeschichtung ein Gemisch von Ethylcellulose und Hydroxypropylmethylcellulose ist.

3. Tablette gemäß Anspruch 1 oder 2, **dadurch gekennzeichnet, dass** die zweite Filmbeschichtung 50-90% Ethylcellulose und 10-50% Hydroxypropylmethylcellulose enthält.

4. Tablette gemäß einem der Ansprüche 1 bis 3, **dadurch gekennzeichnet, dass** sich eine Zwischenbeschichtung zwischen der ersten und der zweiten Beschichtung befindet.

5. Tablette gemäß Anspruch 4, **dadurch gekennzeichnet, dass** die Zwischenbeschichtung aus Polyvinylpyrrolidon besteht.

6. Tablette gemäß einem der Ansprüche 1 bis 5, **dadurch gekennzeichnet, dass** die erste Beschichtung im Wesentlichen aus wenigstens einem Terpolymer besteht, in dem ein wasserlösliches porenbildendes Mittel dispergiert ist.

7. Tablette gemäß einem der Ansprüche 1 bis 6, **dadurch gekennzeichnet, dass** die erste Beschichtung im Wesentlichen aus einem Terpolymer von Vinylchlorid, Vinylacetat und Vinylalkohol besteht.

8. Tablette gemäß einem der Ansprüche 1 bis 7, **dadurch gekennzeichnet, dass** der Wirkstoff im Kern aus der Gruppe ausgewählt ist, die aus Tranquilizern, Antibiotika, Hypnotika, Antihypertonika, Antiangina-Wirkstoffen, Analgetika, entzündungshemmenden Mitteln, Neuroleptika, Antidiabetika, Diuretika, Anticholinergika, Antazida oder Antiepileptika besteht.

9. Tablette gemäß einem der Ansprüche 1 bis 8, **dadurch gekennzeichnet, dass** es sich bei dem Wirkstoff im Kern um Kaliumchlorid, Theophyllin, ein Theophyllinsalz, Phenylpropanolamin, Natriumsalicylat, Cholintheophyllinat, Paracetamol, Carbidopa, Levodopa, Diltiazem, Enalapril, Verapamil, Naproxen, Pseudoephedrin, Nicorandil, Oxybutuin, Morphin, Oxycodon oder Propranolol handelt.

10. Tablette gemäß einem der Ansprüche 1 bis 9, **dadurch gekennzeichnet, dass** es sich bei dem Wirkstoff im Kern um Naproxen handelt.

11. Verfahren zur Herstellung der Tablette gemäß Anspruch 1, das die folgenden Schritte umfasst:
a) Auflösen des Beschichtungs- oder Membranpolymers in einem Lösungsmittel;
b) Herstellen einer Suspension des porenbildenden Materials;
c) Auflösen des Zwischenbeschichtungsmittels in einem Lösungsmittel;
d) Auflösen des Filmpolymers in einem Lösungsmittel;
e) Bereitstellen einer pharmazeutischen Tablette und Kombinieren der Suspension des porenbildenden Materials mit der Lösung des Beschichtungs- oder Membranpolymers unter Bildung einer Beschichtungsflüssigkeit;
f) Auftragen der Beschichtungsflüssigkeit in Form einer Suspension auf die Tablette und Auftrocknen der Beschichtungsflüssigkeit auf die Tablette;
g) Auftragen des aufgelösten Zwischenbeschichtungsmittels auf die Tablette und Trocknen der Tablette;
h) Auftragen des aufgelösten Filmpolymers auf die beschichtete Tablette und Trocknen der Tablette unter Bildung einer beschichteten Tablette mit einem wasserlöslichen porenbildenden Material, das statistisch innerhalb des ersten Beschichtungs- oder Membranpolymers verteilt ist und von einer Zwischenbeschichtung und einer zweiten Filmpolymerbeschichtung bedeckt ist.

12. Verwendung eines hydrophilen Films als zweite Tablettenbeschichtung zur Beseitigung oder Minimierung von Störungen durch die Nahrung auf einer pharmazeutischen Tablette, die einen wirkstoffhaltigen Tablettenkern umfasst, der von einer ersten Beschichtung oder Membran, die für eine gezielte Freisetzung des Wirkstoffs mit im Wesentlichen nullter Ordnung sorgt, umgeben ist, wobei die Beschichtung oder Membran im Wesentlichen aus einem wasserunlöslichen Polymer besteht, in dem feine wasserlösliche Teilchen statistisch verteilt sind.

## Revendications

1. Comprimé pharmaceutique comprenant un noyau de comprimé contenant un médicament enrobé d'un premier enrobage ou d'une membrane permettant d'obtenir une libération prolongée du médicament essentiellement d'ordre zéro, ledit enrobage ou ladite membrane étant essentiellement constituée d'un polymère insoluble dans l'eau dans lequel sont réparties de façon aléatoire des particules fines hydrosolubles, et un deuxième enrobage, **caractérisé en ce que** le deuxième enrobage est un enrobage en film hydrophile, constitué essentiellement d'un polymère insoluble dans l'eau acceptable sur le plan pharmaceutique choisi parmi l'éthylcellulose, la méthylcellulose, le polyméthacrylate, le poly(acétate de vinyle), le poly(acétate de vinyle-co-acide crotonique), le poly(acétate de vinyle-coalcool vinylique), le poly(acrylate d'éthyle, méthacrylate de méthyle, chlorure de méthacrylate de triméthylammonioéthyle) et un polymère hydrosoluble acceptable sur le plan pharmaceutique choisi parmi la gomme guar, l'hydroxyéthylcellulose, l'hydroxypropylcellulose, l'hydroxypropylméthylcellulose, le poly(oxyde d'éthylène), le poly(alcool vinylique), la polyvinylpyrrolidone, aux fins d'éliminer ou de minimiser les interférences alimentaires.

2. Comprimé selon la revendication 1, **caractérisé en ce que** le deuxième enrobage en film est un mélange d'éthylcellulose et d'hydroxypropylméthylcellulose.

3. Comprimé selon la revendication 1 ou 2, **caractérisé en ce que** le deuxième enrobage en film contient 50 à 90% d'éthylcellulose et 10 à 50% d'hydroxypropylméthylcellulose.

4. Comprimé selon l'une quelconque des revendications 1 à 3, **caractérisé en ce qu'**un sous-enrobage est prévu entre le premier et le deuxième enrobage.

5. Comprimé selon la revendication 4, **caractérisé en ce que** le sous-enrobage est constitué de polyvinylpyrrolidone.

6. Comprimé selon l'une quelconque des revendications 1 à 5, **caractérisé en ce que** le premier enrobage est constitué essentiellement d'au moins un terpolymère dans lequel est dispersé un agent hydrosoluble porogène.

7. Comprimé selon l'une quelconque des revendications 1 à 6, **caractérisé en ce que** le premier enrobage est constitué essentiellement d'un terpolymère de chlorure de vinyle, d'acétate de vinyle et d'alcool vinylique.

8. Comprimé selon l'une quelconque des revendications 1 à 7, **caractérisé en ce que** le médicament à l'intérieur du noyau est choisi dans le groupe constitué par les tranquillisants, les antibiotiques, les somnifères, les antihypertenseurs, les antiangineux, les analgésiques, les anti-inflammatoires, les neuroleptiques, les antidiabétiques, les diurétiques, les anticholinergiques, les anti-hyperacidiques ou les anti-épileptiques.

9. Comprimé selon l'une quelconque des revendications 1 à 8, **caractérisé en ce que** le médicament à l'intérieur du noyau est le chlorure de potassium, la théophylline, un sel de théophylline, la phénylpropanolamine, le salicylate de sodium, le théophyllinate de choline, le paracétamol, la carbidopa, la lévodopa, le diltiazem, l'énalapril, le vérapamil, le naproxène, la pseudoéphédrine, le nicorandil, l'oxybutuine, la morphine, l'oxycodone ou le propranolol.

10. Comprimé selon l'une quelconque des revendications 1 à 9, **caractérisé en ce que** le médicament à l'intérieur du noyau est le naproxène.

11. Procédé de préparation du comprimé selon la revendication 1, comprenant les étapes consistant à :
a) dissoudre le polymère d'enrobage ou de membrane dans un solvant ;
b) préparer une suspension de la matière porogène ;
c) dissoudre l'agent de sous-enrobage dans un solvant ;
d) dissoudre le polymère de film dans un solvant ;
e) produire un comprimé pharmaceutique, combiner la suspension de matière porogène et la solution du polymère d'enrobage ou de membrane pour former un fluide d'enrobage ;
f) appliquer le fluide d'enrobage sous la forme d'une suspension sur le comprimé et sécher le fluide d'enrobage sur le comprimé ;
g) appliquer l'agent de sous-enrobage dissous sur le comprimé et sécher le comprimé ;
h) appliquer le polymère de film dissous sur le comprimé enrobé et sécher le comprimé,
pour produire un comprimé enrobé présentant une matière hydrosoluble porogène répartie de façon aléatoire à l'intérieur du polymère de premier enrobage ou de membrane, recouvert d'un sous-enrobage et d'un deuxième enrobage de polymère de film.

12. Utilisation d'un film hydrophile en tant que deuxième enrobage de comprimé pour éliminer ou minimiser les interférences alimentaires d'un comprimé pharmaceutique comprenant un noyau de comprimé contenant un médicament enrobé d'un premier enrobage ou membrane permettant d'obtenir une libération prolongée du médicament essentiellement d'ordre zéro, ledit enrobage ou ladite membrane étant constitué essentiellement d'un polymère insoluble dans l'eau dans lequel sont réparties de façon aléatoire des particules fines hydrosolubles.
